# EUROPEAN PATENT SPECIFICATION

(11) **EP 3 160 557 B1**
(45) Date of publication and mention of the grant of the patent: **12.08.2020**
(21) Application number: 15733892.2
(22) Date of filing: 19.06.2015
(51) Int. Cl.: A61M 15/00, B65D 83/06

(54) **A POWDER INHALER DEVICE**
PULVERINHALATOR
INHALATEUR À POUDRE

(30) Priority: 27.06.2014 IT RE20140057
(43) Date of publication of application: 03.05.2017
(73) Proprietor: Belforti, Sergio, 43036 Fidenza (PR) (IT)
(72) Inventor: Belforti, Sergio, 43036 Fidenza (PR) (IT)
(74) Representative: Corradini, Corrado
(86) International application number: PCT/IB2015/001016
(87) International publication number: WO 2015/198121

(56) References cited:
- EP-A1- 0 404 454
- WO-A1-97/05918
- WO-A2-03/103563
- US-A- 6 098 619

## Description

### TECHNICAL FIELD

The present invention relates to dry powder inhalers, i.e. medical devices which enable administering a precise dose of a medicinal substance in powder form to a patient by inhalation.

### PRIOR ART

Known inhaler devices generally comprise a body provided with a housing able to receive the dose of medicine, to which a mouthpiece is applied. The dose of medicine is received in a housing located on a pathway of a channel opening into the mouthpiece and crossing the body, and sets the mouthpiece in communication with the outside environment.

The air flow crossing the channel at each inhalation of the patient creates a turbulence in the housing which draws the medicine into the mouthpiece and into the patient's airways.

Known inhaler devices are of various types.

Single-dose devices are known, in which one dose of medicine at a time is introduced; the dose is contained in a capsule which is positioned in the housing provided in the inhaler device and then pierced so as to enable the powder to be drawn.

Also known are multi-dose appliances able to receive a plurality of capsules, for example realised in a blister strip, which are placed in the housing of the inhaler one at a time.

Further inhalers are known that can contain a certain quantity of medicine in powder form, which comprise a device for dosing the inhaled powder upon each use of the inhaler.

Document WO 2007/129128 illustrates an inhaler of known type which can be supplied with single single-dose capsules.

WO03/103563A2 discloses the features of the preamble of claim 1.

The known inhalers of the above-indicated types exhibit a series of drawbacks.

Firstly they require a considerable ability of the patient, who on each application must predispose the capsules for use by introducing the medicine and predisposing the capsule which contains the medicine for dispensing. The patient also has to become used to inhaling a flow of air that is specific for each type of apparatus, generally comprised between 30 l/min and 60 l/min, and if the user then changes inhaler, she or he must also adapt to the requirements of the new inhaler.

Further, these inhalers exhibit problems of a hygienic nature, as the mouthpiece must be kept sterile between one application and another, and further require a complete hygienisation when the nature of the substance to be inhaled changes.

There exists a wide variety of inhalers and many pharmaceutical substances are available in one model only.

The patient often has to assume at least two pharmaceutical substances of different nature, using two different inhalers, each of which is characterised by an optimal flow of the inhaled air.

Therefore a patient might be prescribed more than one model with different instructions. The confusion that arises between different models and instructions generally leads to a therapy that is not optimal.

The aim of the invention is to provide an inhaler device which is free of the drawbacks of the prior art, with a solution that is simple and economical.

The aim is attained by an inhaler device having the characteristics set down in the independent claim.

The dependent claims relate to subordinate characteristics aimed at improving the efficiency of the invention.

### DISCLOSURE OF THE INVENTION

The device of the invention is a disposable single-use device that is able to dispense a single dose.

The device can be fashioned in a single piece from a sheet of synthetic material that is hot-profiled, and exhibits a conformation that is such as to make it applicable to the patient's mouth, as a mouthpiece.

Alternatively it can be made in a single piece using an injection process of a synthetic material.

The device also functions as a container of the dose to be dispensed.

In greater detail the device exhibits a truncoconical cavity having an elliptical section, which at the smaller side comprises the containing housing of the pharmaceutical substance, and at the larger base comprises a profiled edge which facilitates the application of the device to the patient's mouth.

The smaller base is thus closed by the housing containing the pharmaceutical substance, while the larger base is open.

At least two oppositely-located holes are present on the wall of the truncoconical surface, which place the inside of the cavity in communication with the outside.

### BRIEF DESCRIPTION OF THE DRAWINGS

The constructional and functional advantages and characteristics of the invention will emerge from the detailed description that follows, which with the aid of the accompanying tables of the drawings illustrates a particular preferred embodiment given by way of non-limiting example.
Figure 1 shows the device seen in plan view.
Figure 2 is section II-II of figure 1.
Figure 3 is section III-III of figure 1.
Figure 4 is a second embodiment of the invention.
Figure 5 is section V-V of figure 4.
Figure 6 is section VI-VI of figure 4.

### PREFERRED EMBODIMENT OF THE INVENTION

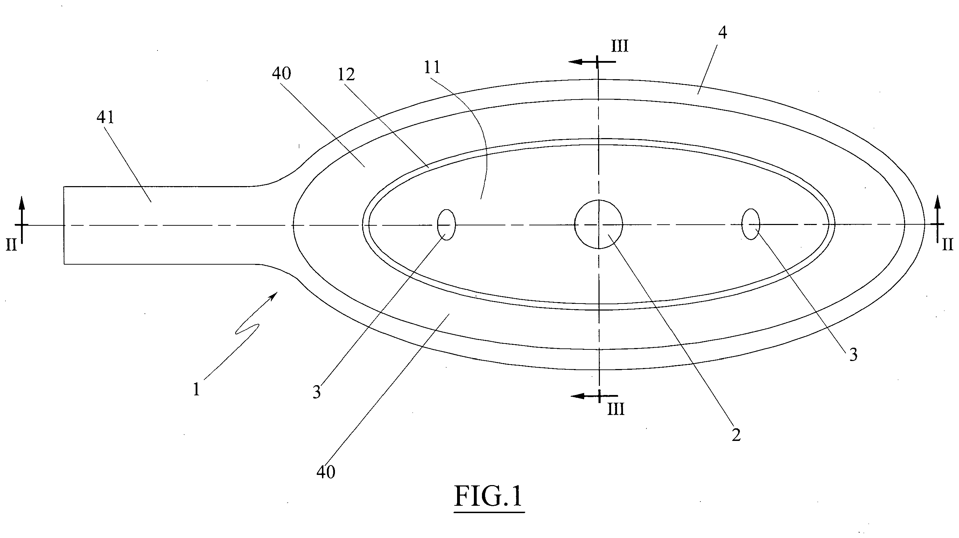

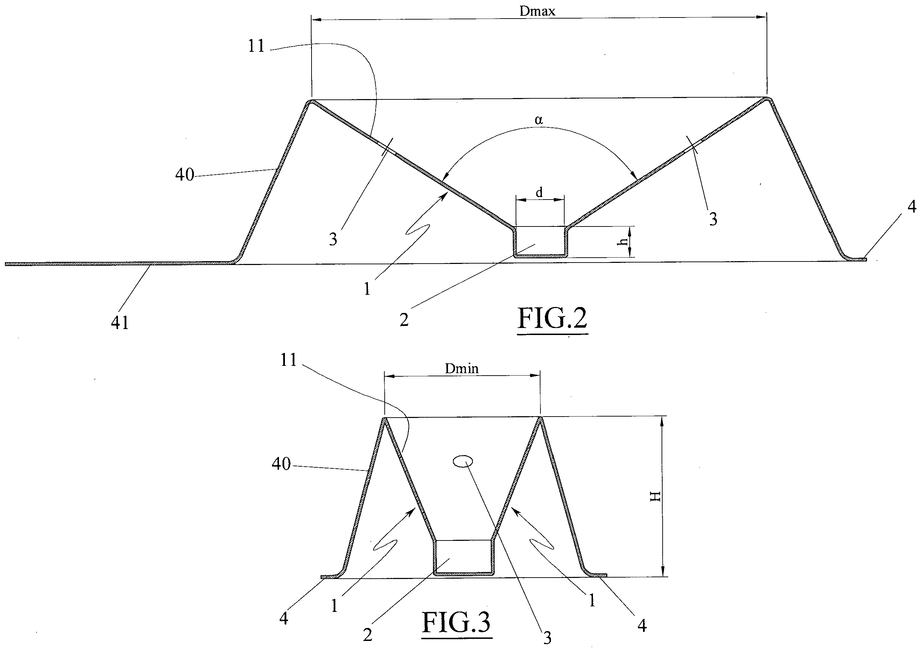

Figures 1 and 3 illustrate a truncoconical body 1 having a slim wall, which exhibits a portion 11 at the vertex of which a cylindrical housing 2 containing the pharmaceutical substance is located.

In plan view 1 the body exhibits an elliptical shape.

Two symmetrical holes 3 are located at the larger axis of the ellipse, the holes 3 having a diameter comprised between 1 mm and 3 mm, and the axis converging towards the open end of the body 1.

The edge 12 of the body 1 bends in a downwards direction to form a profiled mouth shaped so as to fit a user patient's mouth.

The part 40 bent downwards terminates with an edge 4 that is parallel to the mouth of the body 1, facing in an external direction.

The edge 4 is prolonged, at an end and in the direction of the larger axis, into an appendage 41, which enables easy gripping of the device during use thereof.

In the illustrated example the dimensions of the device are the following:
maximum diameter Dₘₐₓ of the larger base of at least 20 mm;
minimum diameter Dₘᵢₙ of the smaller base of at least 10 mm;
height H of at least 10 mm;
diameter d of the housing 2 of at least 5 mm;
height h of the housing 2 of at least 3 mm;
diameter Ø of the holes 3 of at least 1 mm.

The above measurements can obviously vary around the cited amounts.

The angle α of maximum aperture of the truncated cone is comprised between 110° degrees and 130°, preferably 120°.

The device can be fashioned from a single sheet of non-toxic synthetic material by means of heat-forming.

The above-described device can receive a single-dose quantity of medication in powder form in the housing 2.

It can therefore be closed both superiorly and inferiorly by a film adhering to the upper edge of the cylindrical cavity and to the base thereof using known and usual methods.

Thus it functions as a packaging for the medication.

At the moment of use, the patient removes the upper film and applies the hollow body to the mouth, and by aspirating generates a flow of air through the holes 3; the flow creates, internally of the hollow body, a turbulence which atomises the medication and draws it into the airways of the patient.

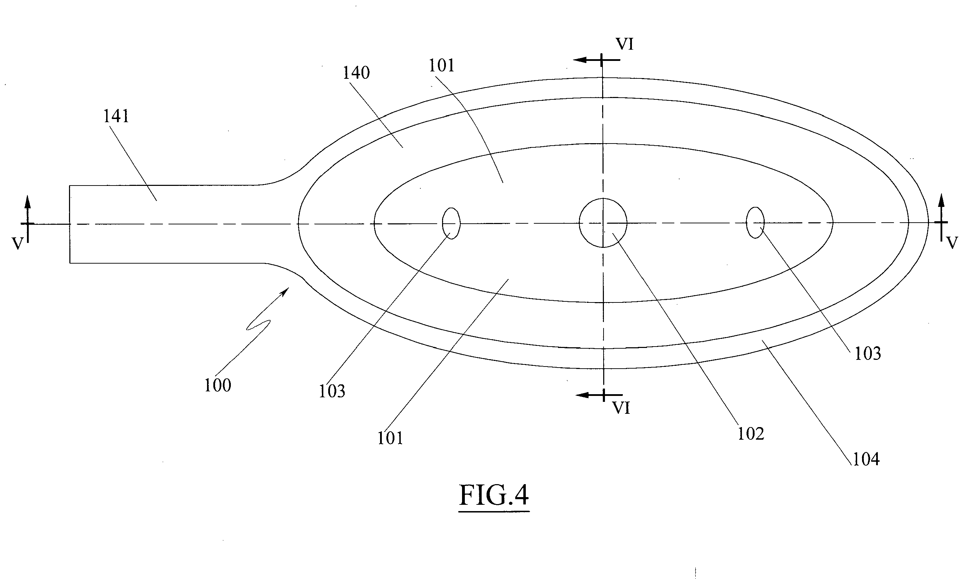

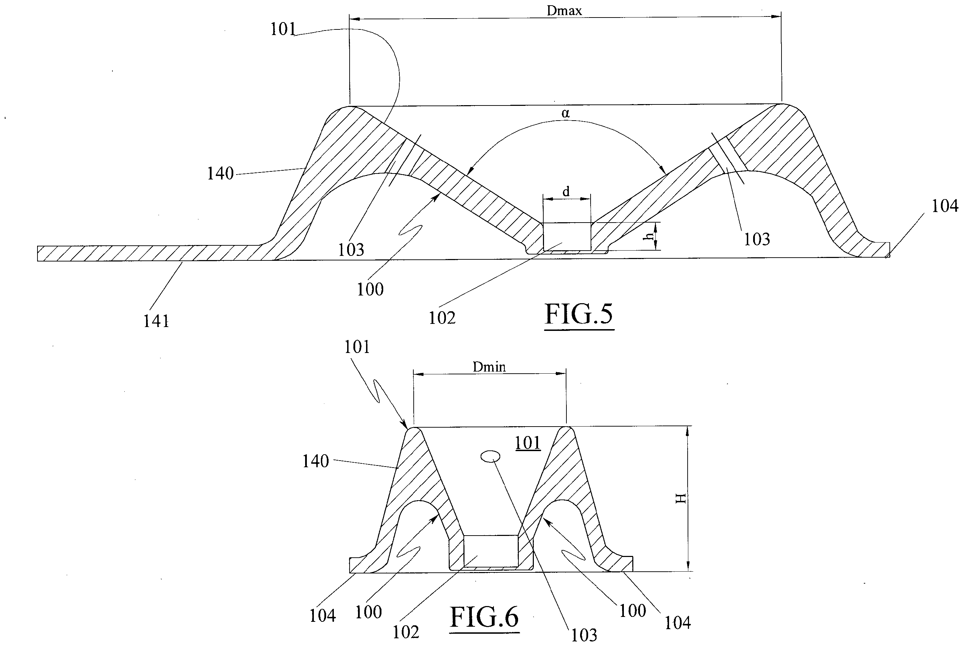

Figures 4 and 6 illustrate a second embodiment of the invention.

A monolithic body 100, fashioned by injection of a non-toxic plastic material, comprises a hollow upper portion 101, profiled as a truncated cone exhibiting an elliptical section having an angle at the vertex α comprised between 110° degrees and 130° degrees, preferably 120° degrees.

The smaller base of the truncoconical hollow portion exhibits a cylindrical housing 102 for containing the pharmaceutical substance.

The body 100 has a flat appendage 141 functioning as a handle and a flat edge 104 coplanar with the handle.

The maximum diameter Dₘₐₓ of the larger base is at least 20 mm, the minimum diameter Dₘᵢₙ of the smaller base is at least 10 mm, the height H is at least 10 mm, the diameter d of the housing 2 is at least 5 mm, the height h of the housing is at least 3 mm.

The above measurements can obviously vary around the cited amounts. The upper cylindrical portion 101 comprises two holes 103 facing and symmetrical and located at the diameter plane comprising the larger axis of the elliptical section, and lie internally of the medial third of the height of the truncoconical cavity.

The holes have the axis thereof inclined towards the larger base, so that when the patient aspirates via the open end of the device, an air-flow is created that is atomized and drawn towards the patient's airways.

The diameter Ø of the holes 103 is at least 1 mm.

The invention is not limited to the embodiment described in the foregoing, and variants and improvements can be brought thereto without forsaking the protective scope of the following claims.

## Claims

1. A powder inhaler device comprising a body (1, 100) provided with a housing (2, 102) able to receive a dose of a medicinal substance, a channelling tangential to the housing and provided with holes (3, 103) communicating with an external environment, a mouthpiece communicating with the channelling and able to aspirate air from the external environment through the holes (3, 103), generating a flow which on a pathway thereof involves the containing housing of the medicine, **characterised in that** the channeling comprises a truncoconical portion (11, 101) of elliptical section at a vertex of which a cylindrical housing (2, 102) containing the pharmaceutical substance is located, and at the opposite open end of which the edge of the body (1, 100) bends in a downwards direction to form a profiled mouth portion shaped so as to fit a user patient's mouth, wherein edges (41, 104) of the profiled mouth portion are bent in an external direction so as to form a flat edge of the body (1, 100) at a same height as the base of the cylindrical housing (2, 102), two symmetrical opposite located holes (3, 103) being afforded on a wall of the truncoconical portion (11, 101), at a larger-diameter plane thereof, which are open to the external environment in order to place the inside of the cavity truncoconical portion (11, 101) in communication with the external environment, and wherein the body (1, 100) provided with the cylindrical housing (2, 102) and the profiled mouth portion shaped so as to fit a user patient's mouth form a disposable single piece unit intended for containing a single dose of the pharmaceutical substance.

2. The device of claim 1, **characterised in that** the maximum diameter Dₘₐₓ of the larger base of the hollow truncoconical portion (11, 101) has a dimension of at least 20 mm, while the minimum diameter Dₘᵢₙ of the larger base of the hollow truncoconical portion (11, 101) has a dimension of at least 10 mm.

3. The device of claim 1, **characterised in that** a height H of the hollow truncoconical portion (11, 101) has a dimension of about 10 mm.

4. The device of claim 1, **characterised in that** the diameter d of the cylindrical housing (2, 102) has a dimension of at least 5 mm.

5. The device of claim 1, **characterised in that** the height h of the cylindrical housing (2, 102) has a dimension of at least 3 mm.

6. The device of claim 1, **characterised in that** the diameter Ø of the holes (3, 103) on the wall of the truncoconical portion (11, 101) has a dimension of at least 1 mm.

7. The device of claim 1, **characterised in that** the angle α of maximum aperture of the truncoconical portion (11,101) is comprised between 110° and 130°, preferably 120° degrees.

## Patentansprüche

1. Pulverinhalatorvorrichtung, umfassend einen Körper (1, 100), der mit einem Gehäuse (2, 102) versehen ist, das eine Dosis einer medizinischen Substanz aufnehmen kann, einen Kanal, der tangential zum Gehäuse verläuft und mit Löchern (3, 103) versehen ist, die mit einer Außenumgebung in Verbindung stehen, ein Mundstück, das mit dem Kanal in Verbindung steht und Luft aus der Außenumgebung durch die Löcher (3, 103) ansaugen kann, wobei ein Strom erzeugt wird, der auf einem Weg desselben das aufnehmende Gehäuse des Medikaments mit einbezieht, **dadurch gekennzeichnet, dass** der Kanal einen kegelstumpfförmigen Abschnitt (11, 101) mit elliptischem Querschnitt umfasst, an dessen Scheitelpunkt sich ein zylindrisches Gehäuse (2, 102) befindet, das die pharmazeutische Substanz enthält, und an dessen gegenüberliegendem offenen Ende sich der Rand des Körpers (1, 100) nach unten biegt, um einen profilierten Mundabschnitt zu bilden, der so geformt ist, dass er in den Mund eines anwendenden Patienten passt, wobei Ränder (41, 104) des profilierten Mundabschnitts in eine äußere Richtung so gebogen sind, dass sie einen flachen Rand des Körpers (1, 100) auf der gleichen Höhe wie die Grundfläche des zylindrischen Gehäuses (2, 102) bilden, wobei zwei symmetrisch gegenüberliegend angeordnete Löcher (3, 103) in einer Wand des kegelstumpfförmigen Abschnitts (11, 101) in einer Ebene mit größerem Durchmesser davon vorgesehen sind, die zur Außenumgebung hin offen sind, um das Innere des kegelstumpfförmigen Hohlraumabschnitts (11, 101) in Verbindung mit der Außenumgebung zu bringen, und wobei der mit dem zylindrischen Gehäuse (2, 102) versehene Körper (1, 100) und der profilierte Mundabschnitt, der so geformt ist, dass er in den Mund eines anwendenden Patienten passt, eine einstückige Einwegeinheit bilden, die zur Aufnahme einer Einzeldosis der pharmazeutischen Substanz bestimmt ist.

2. Vorrichtung gemäß Anspruch 1, **dadurch gekennzeichnet, dass** der maximale Durchmesser Dₘₐₓ der größeren Grundfläche des hohlen, kegelstumpfförmigen Abschnitts (11, 101) eine Abmessung von mindestens 20 mm aufweist, während der minimale Durchmesser Dₘᵢₙ der größeren Grundfläche des hohlen, kegelstumpfförmigen Abschnitts (11, 101) eine Abmessung von mindestens 10 mm aufweist.

3. Vorrichtung gemäß Anspruch 1, **dadurch gekennzeichnet, dass** eine Höhe H des hohlen kegelstumpfförmigen Abschnitts (11, 101) eine Abmessung von etwa 10 mm aufweist.

4. Vorrichtung gemäß Anspruch 1, **dadurch gekennzeichnet, dass** der Durchmesser d des zylindrischen Gehäuses (2, 102) eine Abmessung von mindestens 5 mm aufweist.

5. Vorrichtung gemäß Anspruch 1, **dadurch gekennzeichnet, dass** die Höhe h des zylindrischen Gehäuses (2, 102) eine Abmessung von mindestens 3 mm aufweist.

6. Vorrichtung gemäß Anspruch 1, **dadurch gekennzeichnet, dass** der Durchmesser Ø der Löcher (3, 103) an der Wand des kegelstumpfförmigen Abschnitts (11, 101) eine Abmessung von mindestens 1 mm aufweist.

7. Vorrichtung gemäß Anspruch 1, **dadurch gekennzeichnet, dass** der Winkel α der maximalen Öffnung des kegelstumpfförmigen Abschnitts (11, 101) zwischen 110° und 130°, vorzugsweise 120° Grad, umfasst.

## Revendications

1. Dispositif pour l'inhalation de poudre comprenant un corps (1, 100) pourvu d'un logement (2, 102) apte à recevoir une dose d'une substance médicinale, d'un conduit tangent au logement et pourvu de trous (3, 103) communiquant avec le milieu extérieur, d'un embout buccal communiquant avec le conduit et apte à aspirer de l'air du milieu extérieur à travers les trous (3, 103), générant un flux dont la trajectoire comprend le logement contenant le médicament, **caractérisé en ce que** le conduit comprend une partie tronconique (11, 101) de section elliptique au niveau d'un sommet de laquelle se trouve un logement cylindrique (2, 102) contenant la substance pharmaceutique, et au niveau de l'extrémité opposée ouverte de laquelle le bord du corps (1, 100) est courbé dans une direction allant vers le bas de façon à former une partie embouchure profilée présentant une forme telle qu'elle peut être insérée dans la bouche d'un patient et utilisateur, les bords (41, 104) de la partie embouchure profilée étant courbés dans une direction extérieure de façon à former un bord plat du corps (1, 100) à la même hauteur que la base du logement cylindrique (2, 102), deux trous (3, 103) symétriques, disposés de manière opposée, étant formés sur une paroi de la partie tronconique (11, 101), au niveau d'un plan de plus grand diamètre de celle-ci, lesdits trous débouchant sur le milieu extérieur afin de placer l'intérieur de la partie tronconique (11, 101) formant cavité en communication avec le milieu extérieur, et le corps (1, 100) pourvu du logement cylindrique (2, 102) et la partie embouchure profilée présentant une forme telle qu'elle peut être insérée dans la bouche d'un patient et utilisateur constituant une unité d'un seul tenant jetable prévue pour contenir une dose unique de la substance pharmaceutique.

2. Dispositif selon la revendication 1, **caractérisé en ce que** le diamètre maximal Dₘₐₓ de la base plus large de la partie tronconique (11, 101) creuse présente une dimension d'au moins 20 mm, tandis que le diamètre minimal Dₘᵢₙ de la base plus large de la partie tronconique (11, 101) creuse présente une dimension d'au moins 10 mm.

3. Dispositif selon la revendication 1, **caractérisé en ce qu'**une hauteur H de la partie tronconique (11, 101) creuse présente une dimension d'environ 10 mm.

4. Dispositif selon la revendication 1, **caractérisé en ce que** le diamètre d du logement cylindrique (2, 102) présente une dimension d'au moins 5 mm.

5. Dispositif selon la revendication 1, **caractérisé en ce que** la hauteur h du logement cylindrique (2, 102) présente une dimension d'au moins 3 mm.

6. Dispositif selon la revendication 1, **caractérisé en ce que** le diamètre Ø des trous (3, 103) sur la paroi de la partie tronconique (11, 101) présente une dimension d'au moins 1 mm.

7. Dispositif selon la revendication 1, **caractérisé en ce que** l'angle α d'ouverture maximale de la partie tronconique (11, 101) est compris entre 110° et 130°, de préférence est de 120°.
